# EUROPEAN PATENT APPLICATION

(11) **EP 1 142 585 A1**
(43) Date of publication of application: **10.10.2001**
(21) Application number: 00900336.9
(22) Date of filing: 12.01.2000
(51) Int. Cl.: A61K 45/00, A61K 31/661, A61P 27/00, C07F 9/09

(54) **REMEDIES FOR CORNEAL DISORDERS**

(30) Priority: 12.01.1999 JP 542099
(71) Applicant: SANTEN PHARMACEUTICAL CO., LTD., Higashiyodogawa-ku, Osaka-shi, Osaka 533-8651 (JP); Nishida, Teruo, Ube-shi, Yamaguchi 750-0151 (JP)
(72) Inventor: NISHIDA, Teruo, Ube-shi, Yamaguchi 750-0151 (JP); NAKATA, Katsuhiko, Sakurai-shi, Nara 633-0072 (JP); NAKAMURA, Masatsugu, Nara-shi, Nara 631-0074 (JP)
(74) Representative: Peaucelle, Chantal
(86) International application number: JP0000095
(87) International publication number: WO0041729

(57) **Abstract**

The present invention provides therapeutic agents for corneal disorders comprising compounds having effects of activating Rho as active ingredients, for example corneal epithelial migration promoters. The compounds having the effects of activating Rho are exemplified by lysophosphatidic acid and acyl derivatives thereof. The corneal disorders are exemplified by corneal ulcer, corneal erosion, keratitis and dry eye.

## Description

### Technical Field

The present invention relates to therapeutic agents for corneal disorders having effects of promoting corneal epithelial migration and containing as active ingredients compounds having effects of activating Rho such as lysophosphatidic acid and acyl derivatives thereof.

### Background Art

Corneas are a transparent and avascular tissue having a diameter of about 1 cm and thickness of about 1 mm. Transparency of the cornea affects a visual function greatly. Various physiological and biochemical phenomena in the cornea function mainly for the purpose of maintaining the transparency of the cornea.

Corneal epithelial defect caused by various diseases such as corneal ulcer, corneal erosion, keratitis and dry eye is repaired naturally, unless a mixed infection coincides. However, when the repair is delayed for some reasons or the corneal epithelial defect is persisted without repair, such a delay or persistency exerts harmful influences on normal growth of the epithelium, and further constructions and functions of corneal stroma and endothelium are impaired. A conventional method of treating the corneal epithelial defect is based on a passive principle that corneal epithelium migrates naturally to re-cover defect sites by protecting the corneal surface from external stimulation. In recent years, with development of cell biology, a factor which participates in division, migration, adhesion, extension and the like of cells has been elucidated. It is reported that a compound which promotes the migration of corneal epithelium plays an important role in repairing the corneal epithelial defect (Clin. Ophthalm., 46, 738-743 (1992), Jpn. J. Ophthalm. Surg., 5, 719-727 (1992)).

Cells respond to outside signals and change a cytoskeleton and a cell adhesion mechanism greatly to adapt them to an outside environment. Main constituents forming the cytoskeleton have three types of fiber structure: a microfilament made of actin and the like, a microtubule made of tubulin and the like and an intermediate filament made of keratin and the like. These constituents perform higher-order functions such as cell adhesion, cell shape, cytokinesis and formation of cell polarity while relating closely each other.

Among them, an Rho family, which is one of subfamilies of low-molecular weight GTP-binding protein, is considered to control the actin-microfilament cytoskeleton. The Rho family consists of members such as Rho, Rac and Cdc 42 and acts downstream from extracellular signals such as cell growth factors. Recently, a target protein which is specific for Rho was identified, and regulation mechanisms of cell phenomena are going to be clarified such as a regulation mechanism of the cytoskeleton and the adhesion (Experimental Medicine, 16, 1782-1788 (1998)) and a regulation mechanism of cell movement (Experimental Medicine, 16, 2032-2039 (1998)).

On the other hand, lysophosphatidic acid and acyl derivatives thereof are known as compounds which activate Rho specifically (Cell, 70, 389-399 (1992)). Various actions were reported with regard to lysophosphatidic acid and the acyl derivatives thereof. For example, they enhance binding of fibronectin to cells and regulate cell shape (J. Cell Biol., 127, 1447-1459 (1994)). They enhance fibronectin binding to epithelial and endothelial cells in a skin wound (U.S. Pat. No. 5,480,877). They are skin activators with glycosaminoglycan production-accelerating effects, and are useful as cosmetics and external preparations to prevent skin aging (WO 95/35090). They inhibit hyperproliferation of epithelial cells resulting from psoriasis or the like (U.S. Pat. No. 5,565,439). They activate macrophages and inhibit necrosis in tumor cells (U.S. Pat. No. 5,149,527). They inhibit apoptosis and maintain or restore cell functions (WO 98/41213), etc.

In an ophthalmological field, it was reported that lysophosphatidic acid stimulates proliferation of retinal pigment epithelial cells (Curr. Eye Res., 16, 698-702 (1997)), lysophosphatidic acid sensitizes Ca ion influx in cultured lens epithelial cells (Cell. Signal., 9, 609-616 (1997)), corneal injury results in increased production of lysophosphatidic acid and acyl derivatives thereof in aqueous humor, and they promote proliferation of normal keratocytes (Am. J. Physiol., 274, C1065-C1074 (1988)), and the like.

However, there has been no report concerning a relation between Rho and corneal epithelial cells, and it has not been known, of course, whether compounds having effects of activating Rho exhibit effects on a migration mechanism of the corneal epithelium, which is closely related to the repair of the corneal epithelial defect.

As mentioned above, it was a very interesting subject to study effects of the compounds having the effects of activating Rho, which is a low-molecular weight GTP-binding protein participating in the regulation mechanisms of the cell phenomena, on corneal disorders, particularly on the corneal epithelial migration through studies of participation of Rho in the migration mechanism of the corneal epithelium.

In order to study the participation of Rho in the migration mechanism of the corneal epithelium, the present inventors first studied effects of Rho inhibitors on the corneal epithelium. As a result, it was clarified that the corneal epithelial migration is completely inhibited by the Rho inhibitors and regulation of intracellular scafford protein by Rho, which is the low-molecular weight GTP-binding protein, participates in the migration mechanism of the corneal epithelium.

Next, studying effects of the compounds having the Rho-activating effects, the present inventors found that the compounds have excellent effects of promoting the corneal epithelial migration.

Further, when the compounds having the Rho-activating effects and the Rho inhibitors were used jointly, the above-mentioned promotion of the corneal epithelial migration was almost completely inhibited. It was confirmed that the effects of promoting the corneal epithelial migration are based on the Rho-activating effects.

The above-mentioned results clearly show that the compounds having the Rho-activating effects have the excellent effects of promoting the corneal epithelial migration and are useful as therapeutic agents for corneal disorders such as corneal ulcer, corneal erosion, keratitis and dry eye wherein the cornea is injured from various causes.

### Disclosure of the Invention

Compounds having Rho-activating effects in the present invention mean compounds which augment regulation mechanisms of cell phenomena in which Rho participates.

Lysophosphatidic acid and acyl derivatives thereof are compounds represented by the following general formula [I]: 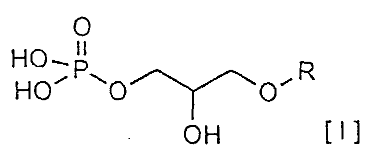 wherein R is hydrogen or acyl.

The acyl in the compounds [I] is saturated or unsaturated aliphatic carbonyl or aromatic carbonyl, preferably saturated or unsaturated aliphatic carbonyl, more preferably higher aliphatic carbonyl having six or more carbon atoms, and particularly preferred examples of the acyl are oleoyl and stearoyl.

Corneal disorders in the present invention are corneal ulcer, corneal erosion, keratitis, dry eye and the like wherein cornea is injured from various causes.

In order to study participation of Rho in a migration mechanism of corneal epithelium, the present inventors studied the effects of Rho inhibitors and the compounds having the Rho-activating effects, on the corneal epithelium. Details are described in a section of "Pharmacological Tests" hereinbelow. Corneal epithelial migration was recognized to be almost completely inhibited by a C3 enzyme, which is an exoenzyme of *Clostridium botulinum* and is known as the Rho inhibitor (hereinafter referred to as "Exoenzyme C3") (Cell, 70, 389-399 (1992)).

This result clearly shows that Rho, which is low-molecular weight GTP-binding protein, participates in the migration mechanism of the corneal epithelium.

Next, studying effects of lysophosphatidic acid and the acyl derivatives thereof, which are typical compounds having the Rho-activating effects, on corneal epithelial migration, it was found that lysophosphatidic acid and the acyl derivatives thereof promote corneal epithelial migration in a corneal piece tissue culture system. Further, it was recognized that such corneal epithelial migration-promoting effects are almost completely inhibited by Exoenzyme C3, and it was confirmed that the corneal epithelial migration-promoting effects of lysophosphatidic acid and the acyl derivatives thereof are based on the Rho-activating effects. These results clearly show that the compounds having the Rho-activating effects have excellent effects of promoting the corneal epithelial migration and are useful for treatment of the corneal disorders, namely corneal ulcer, corneal erosion, keratitis, dry eye and the like wherein the cornea is injured from the various causes.

The compound having the Rho-activating effect can be administered orally or parenterally. Examples of dosage forms are tablets, capsules, granules, powders, injections, eyedrops and the like, and eyedrops are particularly preferable. The compound can be formulated into preparations by general techniques. For example, eyedrops can be prepared by optionally using an isotonic agent such as sodium chloride or concentrated glycerine; a buffer such as sodium phosphate or sodium acetate; a surfactant such as polyoxyethylenesorbitan monooleate, polyoxyl 40 stearate or polyoxyethylene hydrogenated castor oil; a stabilizer such as sodium citrate or disodium edetate; a preservative such as benzalkonium chloride or paraben; or the like. pH can be in a range acceptable for ophthalmic preparations, and it is preferably in a range of 4 to 8. Eye ointments can be prepared by using a general base such as white vaseline or liquid paraffin. Oral preparations such as tablets, capsules, granules and powders can be prepared by optionally adding a diluent such as lactose, crystalline cellulose, starch or vegetable oil; a lubricant such as magnesium stearate or talc; a binder such as hydroxypropylcellulose or polyvinyl pyrrolidone; a disintegrator such as calcium carboxymethylcellulose or low-substituted hydroxypropylmethylcellulose; a coating agent such as hydroxypropylmethylcellulose, macrogol or silicone resin; a film forming agent such as gelatin film; or the like.

The dosage can be selected suitably depending on symptoms, age, dosage form and the like. In the case of eyedrops, they are instilled once to several times per day with a concentration of 0.0001 to 1% (w/v), preferably 0.001 to 1% (w/v) solution. In the case of oral preparations, the usual daily dosage is 0.1 to 5000 mg, preferably 1 to 1000 mg, which can be given in a single dose or several divided doses.

Examples of formulation and results of pharmacological tests are shown below. These examples do not limit the scope of the invention, but are intended to make the invention more clearly understandable.

### Best Mode for Carrying out the Invention

### Formulation

Typical examples of formulation using oleoyl lysophosphatidic acid (hereinafter referred to as "oleoyl LPA") as a compound having an Rho-activating effect are shown below.

### 1. Eyedrops

Eyedrops having the following formulation were prepared by a general method.

| Formulation 1 (ophthalmic solution) | |
|---|---|
| In 100 ml | |
| Oleoyl LPA | 1 mg |
| Sodium chloride | 900 mg |
| Sodium hydroxide | q.s. |
| Hydrochloric acid | q.s. |
| Sterile purified water | q.s. |

Ophthalmic solutions containing 5 mg, 10 mg, 50 mg, 100 mg, 500 mg and 1000 mg of oleoyl LPA in 100 ml can be prepared by optionally adding a surfactant or a stabilizer by a method similar to Formulation 1.

| Formulation 2 (eye ointment) | |
|---|---|
| In 100 g | |
| Oleoyl LPA | 100 mg |
| White vaseline | 90 g |
| Liquid paraffin | q.s. |

Eye ointments containing 1 mg, 5 mg, 10 mg and 50 mg of oleoyl LPA can be prepared by a method similar to Formulation 2.

| Formulation 3 (tablet) | |
|---|---|
| In 100 mg | |
| Oleoyl LPA | 10 mg |
| Lactose | 59.4 mg |
| Cornstarch | 20 mg |
| Calcium carboxymethylcellulose | 6 mg |
| Hydroxypropylcellulose | 4 mg |
| Magnesium stearate | 0.6 mg |

Desired coated tablets can be obtained by coating tablets according to the formulation as above with 2 mg/tablet of a coating agent such as hydroxypropylcellulose.

Tablets containing 0.1 mg, 0.5 mg, 1 mg, 5 mg and 50 mg of oleoyl LPA in 100 mg can be obtained by a method similar to Formulation 3.

### Pharmacological Tests

### Effect on corneal epithelial migration (in vitro)

Effects of the following test compounds on corneal epithelial migration were studied using cornea of a male Japanese white rabbit by using corneal epithelial migration length in a corneal piece tissue culture system as an index according to the method of Nishida et al. (J. Cell Biol., 97, 1653-1657 (1983)).

### Experimental method

A corneal block isolated from a corneal piece of a rabbit was cultured for 24 hours in a culture medium (TC-199) containing the test compound under a condition of 37°C-5% CO₂. After the culture, the corneal block was fixed in a mixed liquid of ethanol-glacial acetic acid (volume ratio 95:5) and embedded with paraffin to prepare a section. Removing the paraffin from the section, the section was stained with hematoxylin-eosin, and migration length of an epithelial cell layer was measured under a microscope.

A corneal block cultured similarly in a culture medium containing no test compound was used as a control.

### Result 1

Table 1 shows a result obtained by culturing the corneal block in a culture medium containing Exoenzyme C3, which is an Rho inhibitor, as the test compound.

**Table 1**

| | Migration length (*µ*m) |
|---|---|
| Control | 454 |
| Exoenzyme C3 (2 *µ* g/ml) | 186 |
| (Each datum in the table is an average of six samples.) | |

Table 1 clearly shows that when the corneal block is cultured in the culture medium containing Exoenzyme C3, which is the Rho inhibitor, the corneal epithelial migration is almost completely inhibited, and Rho participates in the corneal epithelial migration.

### Result 2

Table 2 shows results obtained by culturing the corneal block in a culture medium containing oleoyl LPA as the test compound at concentrations of 0.02 *µ*M, 0.2 *µ*M and 2 *µ*M respectively.

**Table 2**

| | Migration length (*µ*m) |
|---|---|
| Control | 454 |
| Oleoyl LPA (0.02 *µ* M) | 528 |
| (0.2 *µ*M) | 658 |
| (2 *µ*M) | 712 |
| (Each datum in the table is an average of six samples.) | |

Table 2 shows that when the corneal block is cultured in the culture medium containing oleoyl LPA, the corneal epithelial migration is remarkably promoted concentration dependently.

### Result 3

Table 3 shows results obtained by adding Exoenzyme C3 to the culture medium together with oleoyl LPA as the test compounds.

**Table 3**

| | Migration length (*µ* m) |
|---|---|
| Control | 454 |
| Exoenzyme C3 (2 *µ*g/ml) | |
| + Oleoyl LPA (0.02 *µ* M) | 185 |
| + Oleoyl LPA (0.2 *µ*M) | 182 |
| + Oleoyl LPA (2 *µ*M) | 198 |
| (Each datum in the table is an average of six samples.) | |

Table 3 shows that when Exoenzyme C3, which is the Rho inhibitor, is added to the culture medium together with oleoyl LPA, the corneal epithelial migration is almost completely inhibited.

These results confirm that the corneal epithelial migration-promoting effect is based on the Rho-activating effect.

The above-mentioned pharmacological tests show that the compound having the Rho-activating effect has the excellent effects of promoting corneal epithelial migration and is useful as a therapeutic agent for corneal disorders such as corneal ulcer, corneal erosion, keratitis and dry eye wherein the cornea is injured from various causes, through an effect of promoting wound healing of the corneal epithelium.

### Industrial Applicability

The present invention relates to therapeutic agents for corneal disorders containing compounds having Rho-activating effects as active ingredients, for example corneal epithelial migration promoters.

## Claims

1. A therapeutic agent for a corneal disorder comprising a compound having an effect of activating Rho as an active ingredient.

2. The therapeutic agent for the corneal disorder as claimed in claim 1, wherein the compound having the effect of activating Rho is lysophosphatidic acid or an acyl derivative thereof.

3. The therapeutic agent for the corneal disorder as claimed in claim 2, wherein the acyl derivative of lysophosphatidic acid is oleoyl lysophosphatidic acid.

4. The therapeutic agent for the corneal disorder as claimed in any one of claims 1 to 3, wherein the corneal disorder is corneal ulcer, corneal erosion, keratitis or dry eye.

5. A corneal epithelial migration promoter comprising a compound having an effect of activating Rho as an active ingredient.
